# EUROPEAN PATENT SPECIFICATION

(11) **EP 2 866 705 B1**
(45) Date of publication and mention of the grant of the patent: **31.05.2017**
(21) Application number: 12879644.8
(22) Date of filing: 27.06.2012
(51) Int. Cl.: A61B 17/70, A61F 2/44, A61B 17/82, A61B 17/86, A61B 17/00

(54) **PROSTHESIS FOR VERTEBRAL COMPRESSION FRACTURE**
PROTHESE FÜR WIRBELKÖRPER-KOMPRESSIONSFRAKTUR
PROTHÈSE POUR FRACTURE DE COMPRESSION VERTÉBRALE

(43) Date of publication of application: 06.05.2015
(73) Proprietor: Warsaw Orthopedic, Inc., Warsaw, IN 46581 (US)
(72) Inventor: GLASER, Adam D., Germantown, Tennessee 38138 (US); BRAAL, Jaredan, M, Memphis, Tennessee 38115 (US); LIM, Roy K., Germantown, Tennessee 38138 (US); ZHANG, Jeffrey, Collierville, Tennessee 38017 (US)
(74) Representative: Viering, Jentschura & Partner mbB Patent- und Rechtsanwälte
(86) International application number: PCT/US2012/044337
(87) International publication number: WO 2014/003734

(56) References cited:
- US-A- 5 658 284
- US-A- 5 658 284
- US-A- 6 063 089
- US-A- 6 063 089
- US-A1- 2008 215 095
- US-A1- 2009 204 153
- US-A1- 2010 305 613
- US-A1- 2011 218 626
- US-B2- 7 090 674
- US-B2- 7 175 622
- US-B2- 7 678 136
- US-B2- 7 678 136

## Description

### TECHNICAL FIELD

The present disclosure generally relates to medical devices and systems for the treatment of musculoskeletal disorders, and more particularly to a spinal implant fixation system that employs a connector for linear implants to provide stabilization of vertebrae.

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility. For example, after a disc collapse, severe pain and discomfort can occur due to the pressure exerted on nerves and the spinal column.

Non-surgical treatments, such as medication, rehabilitation and exercise can be effective, however, may fail to relieve the symptoms associated with these disorders. Surgical treatments of these spinal disorders include discectomy, laminectomy, fusion and implantable prosthetics. During surgical treatment, one or more rods may be attached via fasteners to the exterior of two or more vertebral members.

Normally when a rod is positioned within a bone screw and affixed therein via a set screw, the rod runs parallel with an axis defined by a channel within the receiver of the bone screw. In order to adjust, however slightly, an angle between the bone screw and the rod, bending of the rod is required to provide the proper lordosis. As rods are generally made of hardened materials, bending rods can often prove difficult. This disclosure describes an improvement over these prior art technologies.

Implant connector systems are known from US 2008/215095 A1, US 2009/204153 A1, US 2010/305613 A1, US 2011/218626 A1, US 7 678 136 B2, and US 5 658 284 A.

### SUMMARY

According to the present invention, an implant connector system according to claim 1 is provided. Examples of such systems are defined in the dependent claims.

The methods not covered by the claims represent background art that is useful for understanding the invention.

A connector of an exemplary system comprises a first member defining a first cavity positioned along a longitudinal axis of the first member and configured to receive an implant, the first member includes a first end and a second end, the first end having a bulbous shape configured to prevent translational movement of the connector along a longitudinal axis of a receiving portion of a bone screw. The connector also comprises a second member connected to the first member along a first axis and defining a second cavity positioned along a longitudinal axis of the second member and transverse to the longitudinal axis of the first member and configured to receive a fixation element to affix the implant within the first member, the second member connected to the first member substantially at the second end.

In one embodiment, an implant connector system comprises an implant connector, comprising a first member having a first end and a second end and defining a first cavity positioned along a longitudinal axis of the first member and configured to receive an implant, the first end having a bulbous shape. The implant connector also includes a second member connected to the first member along a first axis and substantially at the second end and defining a second cavity positioned along a longitudinal axis of the second member and transverse to the longitudinal axis of the first member and configured to receive a fixation element to affix the implant within the first member, the second member connected to the first member substantially at the second end. The implant connector system also includes a bone screw having a receiving portion defining a channel configured to engage with the bulbous shape of the first end of the implant connector to prevent translational movement of the connector along a longitudinal axis of the channel.

In one embodiment not covered by the present invention, a method of anchoring a surgical rod within a bone screw, comprises inserting a first bone screw into tissue; inserting a second bone screw into tissue; inserting a surgical rod into a connector having a first member defining a first cavity positioned along a longitudinal axis of the first member and configured to receive the surgical rod and a second member connected to the first member along a first axis and defining a second cavity positioned along a longitudinal axis of the second member and transverse to the longitudinal axis of the first member and configured to receive a fixation element to affix the surgical rod within the first member; the first member includes a first end and a second end, the second member connected to the first member substantially at the second end, the first end having a bulbous shape configured to prevent translational movement of the connector along a longitudinal axis of a receiving portion of a bone screw; placing a first section of the surgical rod into a receiver of the first bone screw; placing the bulbous shape of the first end of the connector into a receiver of the second bone screw; affixing the surgical rod into the connector; affixing the surgical rod in the receiver of the first bone screw; and affixing the connector in the receiver of the second bone screw.

### BRIEF DESCRIPTION OF THE DRAWINGS

The present disclosure will become more readily apparent from the specific description accompanied by the following drawings, in which:
FIG. 1 is a perspective view of one embodiment of a connector in accordance with the principles of the present disclosure;
FIG. 2 is a top view of the connector shown in FIG. 1;

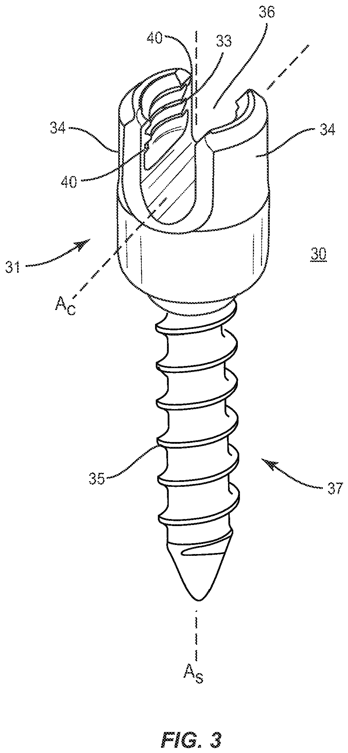

FIG. 3 is a perspective view of a bone screw used with the connector shown in FIG. 1;
FIG. 4 is a top view of the bone screw shown in FIG. 3;
FIG. 5 is a cross sectional view of one embodiment of a connector system in accordance with the principles of the present disclosure;
FIG. 6 is a top view of the connector system shown in FIG. 5;

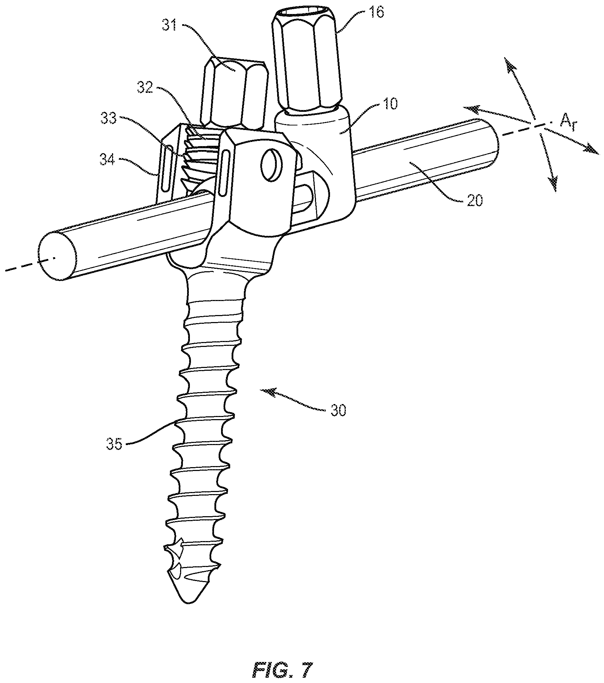

FIG. 7 is a perspective view of the connector system shown in FIG. 5;
FIG. 8 is a perspective view of one embodiment of a connector in accordance with the principles of the present disclosure;
FIG. 9 is a top view of the connector shown in FIG. 8;
FIG. 10 is a side view of the connector shown in FIG. 8;
FIG. 11 is a partial cross sectional view of a connector system in accordance with the principles of the present disclosure;
FIG. 12 is a perspective view of one embodiment of a connector in accordance with the principles of the present disclosure;
FIG. 13 is a top view of the connector shown in FIG. 12;
FIG. 14 is a side view of the connector shown in FIG. 12;
FIG. 15 is a partial cross sectional view of a connector system in accordance with the principles of the present disclosure;
FIG. 16 is a perspective view of one embodiment of a connector in accordance with the principles of the present disclosure;
FIG. 17 is a top view of the connector shown in FIG. 16;
FIG. 18 is a side view of the connector shown in FIG. 16;
FIG. 19 is a perspective view of one embodiment of a connector in accordance with the principles of the present disclosure;
FIG. 20 is a top view of the connector system shown in FIG. 19;
FIG. 21 is a side view of the connector shown in FIG. 19; and
FIG. 22 is a perspective view of a connector system in accordance with the principles of the present disclosure.

Like reference numerals indicate similar parts throughout the figures.

### DETAILED DESCRIPTION

The exemplary embodiments of the connector are discussed in terms of medical devices for the treatment of musculoskeletal disorders and more particularly, in terms of a bone fastener that provides stabilization for treating a vertebral column. It is contemplated that the connector provides a robust system and method for creating proper lordosis in a spinal rod system, for example, by allowing for distraction of a vertebral body along a longitudinal axis of a surgical rod independent of lordotic angle adjustment.

It is envisioned that the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. It is contemplated that the present disclosure may be employed with other osteal and bone related applications, including those associated with diagnostics and therapeutics. It is contemplated that the disclosed connector and system may be alternatively employed in a surgical treatment with a patient in a prone or supine position, and/or employ various surgical approaches to the spine, including anterior, posterior, posterior mid-line, medial, lateral, postero-lateral, and/or antero-lateral approaches, and in other body regions. The present disclosure may also be alternatively employed with procedures for treating the lumbar, cervical, thoracic, sacral and pelvic regions of a spinal column. The connector and system of the present disclosure may also be used on animals, bone models and other non-living substrates, such as, for example, in training, testing and demonstration.

The present disclosure may be understood more readily by reference to the following detailed description of the disclosure taken in connection with the accompanying drawing figures, which form a part of this disclosure. It is to be understood that this disclosure is not limited to the specific devices, conditions or parameters described and/or shown herein, and that the terminology used herein is for the purpose of describing particular embodiments by way of example only and is not intended to be limiting of the claimed disclosure. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "superior" and "inferior" are relative and used only in the context to the other, and are not necessarily "upper" and "lower".

Further, as used in the specification and including the appended claims, "treating" or "treatment" of a disease or condition refers to performing a procedure that may include administering one or more drugs to a patient in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

The components of the connector can be fabricated from biologically acceptable materials suitable for medical applications, including metals, synthetic polymers, ceramics and bone material and/or their composites, depending on the particular application and/or preference of a medical practitioner. For example, the components of the connector and system, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL® manufactured by Toyota Material Incorporated of Japan), ceramics and composites thereof such as calcium phosphate (e.g., SKELITE™ manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations. Various components of the bone fastener system may have material composites, including the above materials, to achieve various desired characteristics such as strength, rigidity, elasticity, compliance, biomechanical performance, durability and radiolucency or imaging preference. The components of the bone fastener system, individually or collectively, may also be fabricated from a heterogeneous material such as a combination of two or more of the above-described materials.

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications in the described devices, instruments, methods, and any further application of the principles of the disclosure as described herein are contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. The following discussion includes a description of a bone fastener system and related methods of employing the bone fastener and system in accordance with the principles of the present disclosure. Alternate embodiments are also disclosed. Reference will now be made in detail to the exemplary embodiments of the present disclosure, which are illustrated in the accompanying figures.

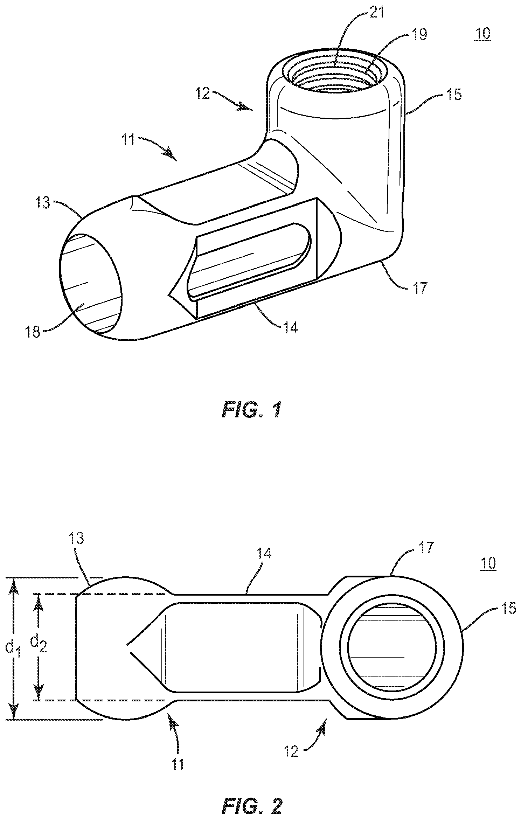

In one embodiment, as shown in FIG. 1, the connector 10 includes a first member 11 attached to a second member 12. First and second members 11, 12 can be mechanically attached to each other or monolithically formed.

First member includes a first end 13, a mid-section 14 and a second end 17. First member 11 is configured to support and retain a linear implant 20, such as, for example, a vertebral or other surgical rod, within a first cavity 18 defined within first member 11. Cavity 18 is generally bore-shaped and opens through both first end 13 and second end 17 of first member 11. Cavity 18 extends along a longitudinal axis of first member 11. First end 13 is spherical in shape and defines a width d1 and mid-section 14 defines a width d2; width d1 is greater than a width d2. As viewed from the top and as illustrated in FIG. 2, first end 13 and mid-section 14 form a bulbous shape. In this embodiment, the bulbous shape acts as an engagement member to lock the connector 10 into a bone anchor receiver.

Second member 12 defines a second cavity 19 extending from a top end 15 and opening into cavity 18. Second member 12 is configured to engage with a fixation element 16 to affix implant 20 within connector 10. Fixation element 16, for example a threaded set screw, includes threads 22 that are configured to engage with threads 21 defined within cavity 19. After implant 20 is placed within cavity 18, fixation element 16 can be tightened down onto implant 20 to secure implant 20 within connector 10. Other types of fixation elements and systems are contemplated.

Connector 10 is designed to engage with bone screw 30. Bone screw 30 is configured to anchor into bone or other tissue. Although a bone screw is used as an example of an anchor, other types of anchors are contemplated. Bone screw 30 includes upper portion 31 and lower portion 37. Lower portion 37 includes threads 35 for engaging with bone or other tissue. Longitudinal axis As is defined along bone screw 30. Upper portion 31 includes opposing arms 34 defining a channel 36 that runs along a longitudinal axis Ac of channel 36. Upper portion 31 also includes threads 33 defined on arms 34. Threads 33 are configured to engage with threads 32 of a fixation element 31. Although fixation element 31 is shown as a threaded set screw, other fixation elements and systems are contemplated. In addition, although bone screw 30 is depicted as a top loading anchor, other variations such as side loading anchors are contemplated.

Channel 36 includes a circular space 38 and two opposing spaces 39. Arms 34 define circular space 38 between the arcuate portions of threads 33. Inner edges 40 of arms 34 define opposing spaces 39. A width d3 is defined between the inner edges of threads 33 of opposing arms 34 and represents a diameter of circular space 38. Circular space 38 is of a size and shape to fit first end 13 therein. The engagement member of first end 13 is shaped such that the first end is retained within the circular space 38 of the channel 36 as described below. Other shapes besides the circular shape 38 and bulbous shape of the first end 13 may be mated accordingly such that the combination fixes translational movement between the anchor and the connector 10. A width d4 is the distance between inner edges 40 of arms 34. An opposing space 39 is of a size and shape to fit mid-section 14 therein. Width d3 is greater than width d4.

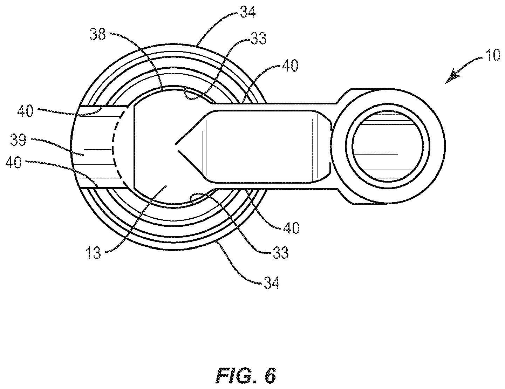

Channel 36 is defined such that the bulbous shape of first end 13 fits within circular space 38 of channel 36, that is, the arcuate shape of first end 13 is similar to the arcuate shape of threads 33. In addition, channel 36 is defined such that mid-section 14 fits within one of opposing spaces 39 of channel 36. When mated, as shown in FIG. 6, first end 13 and mid-section 14 fit within circular space 38 and an opposing space 39, respectively. Width d1 of first end 13 is less than width d3 to allow first end to fit within channel 36, but is greater than width d4, which prevents connector 10 from any lateral movement along axis Ac after connector 10 is mated with bone screw 30.

Bone screw 30 and connector 10 are configured such that when connector 10 is engaged with bone screw 30 in channel 36, connector 10 is rotatable within channel 36 due to the spherical shape of first end 13, but cannot translate along axis Ac. When implant 20 is engaged with connector 10 and connector 10 is engaged with bone screw 30, implant 20 can rotate with respect to axis Ac to adjust an angle between axis Ac and axis Ar, thus providing a proper lordotic angle without the need to bend implant 20.

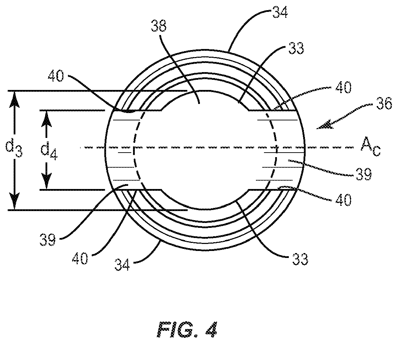

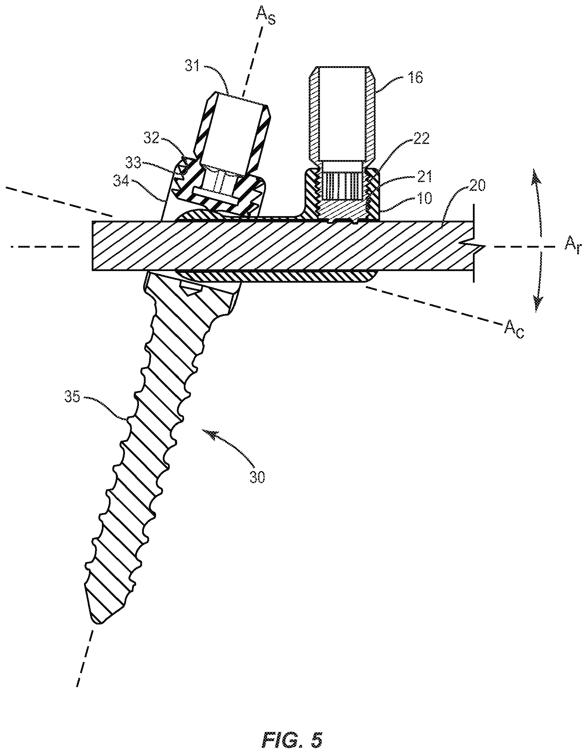

In operation, implant 20 is positioned within connector 10. Connector 10 is then positioned within channel 36. After connector 10 is adjusted to a required position and angle between axes Ac and Ar, implant 20 can be affixed in connector 10 by tightening fixation element 16 onto implant 20 through channel 19. Connector 10 can be affixed into position by tightening fixation element 31 onto first end 13. Due to the configuration of the bulbous shape of the first end 13 and circular space 38 of channel 36, connector 10 will remain within channel 36 as fixation element is tightened. Although the rotation is described in the directions shown in FIG. 4, rotation is also available in a side-to-side manner as shown in FIG. 5. By moving connector 10 within channel 36, the angle at which implant 20 is positioned relative to axis As can be varied to suit the particular needs of a patient, without the difficult task of bending implant 20.

Normally, the downward force of a bone screw fixation element onto a known connector would force the connector to translate out of a bone screw along the axis of the channel. With the present invention, the bulbous shape of first end 13 mating with circular space 38 prevents any lateral movement and maintains connector 10 within channel 36.

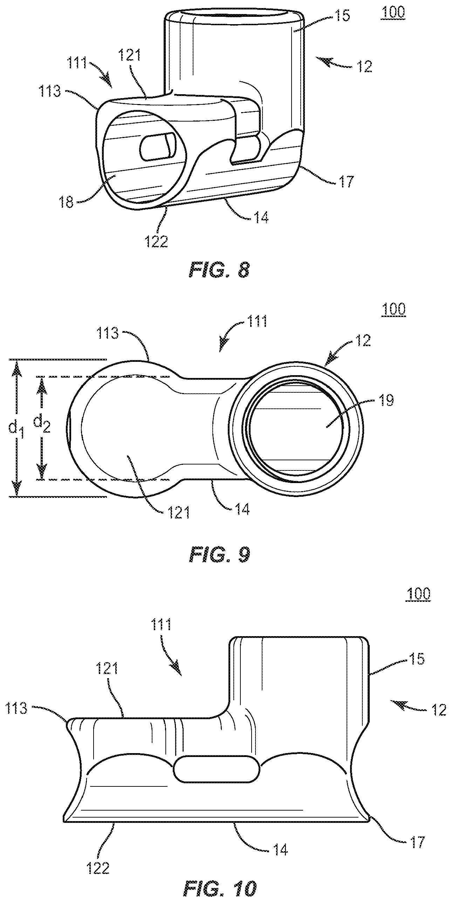

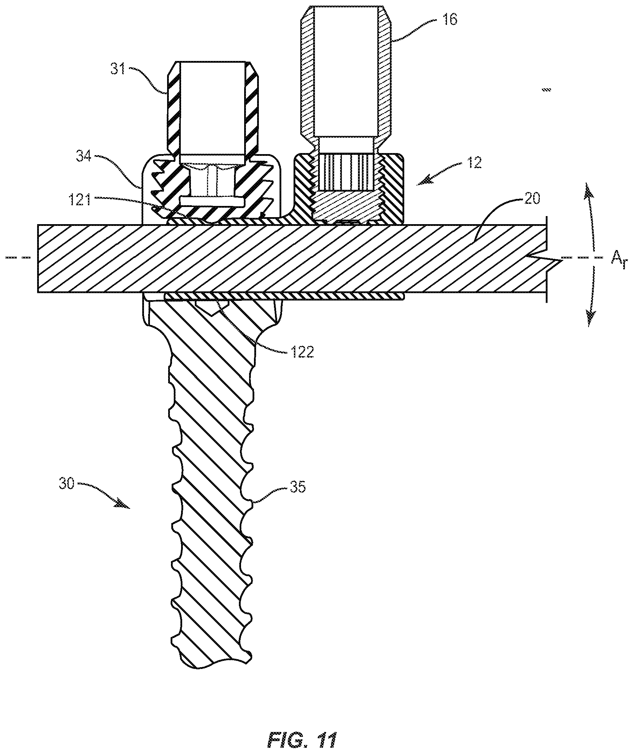

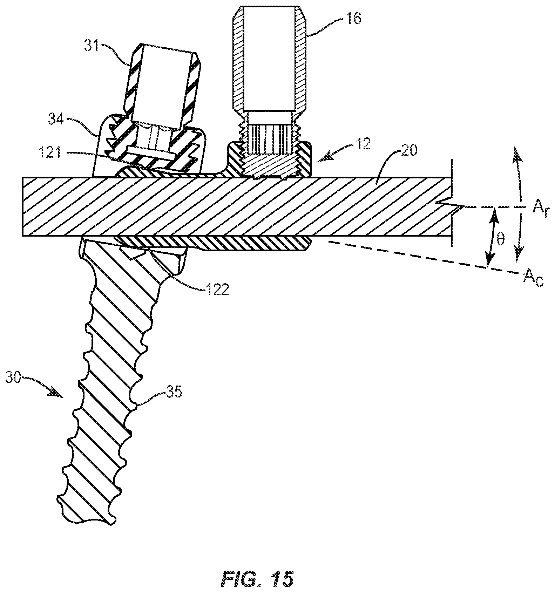

In other embodiments, the connector can provide a fixed lordotic angle. Fixing the lordotic angle can be achieved by providing at least one planar surface to the first end of the first member. These variations will be described with respect to FIGs. 8-22. In each of the variations to be described, the second member, the implant and the bone screw are similar to those described above and will not be described in further detail.

FIGs. 8-11 illustrate a connector 100 having a lordotic angle of 0 degrees, that is, when connector 100 including rod 20 is engaged with bone screw 30 axis Ar is substantially parallel to axis Ac. A first member 111 includes a first end 113 having an upper surface 121 and a lower surface 122. First end 113 maintains a circular shape with width d1 when observed down at an angle transverse to axis Ac. The circular shape of first end 113 is designed to fit within circular space 38 of channel 36 of bone screw 30. Upper surface 121 is planar. Lower surface 122 is arcuate in cross section transverse to axis Ac and configured to mate with a bottom of channel 36. Mid-section 14 defines a width d2.

In operation, implant 20 is positioned within connector 100 and connector 100 is positioned within channel 36, similar to that shown in FIG. 6. The bulbous shape of first end 113 prevents movement along axis Ac of connector 100. Fixation element 16 is tightened onto implant 20 to affix implant 20 in connector 100. Fixation element 31 is tightened onto top surface 121 of connector 100. Since a bottom surface of fixation element 31 is flat and top surface 121 is planar, fixation element 31 will prevent rotation of connector 100 and force bottom surface 122 to securely mate with channel 36 with axis Ar of implant 20 in alignment with axis Ac of channel 36. The bulbous shape of first end 113 mating with circular space 38 prevents any lateral movement and maintains connector 100 within channel 36.

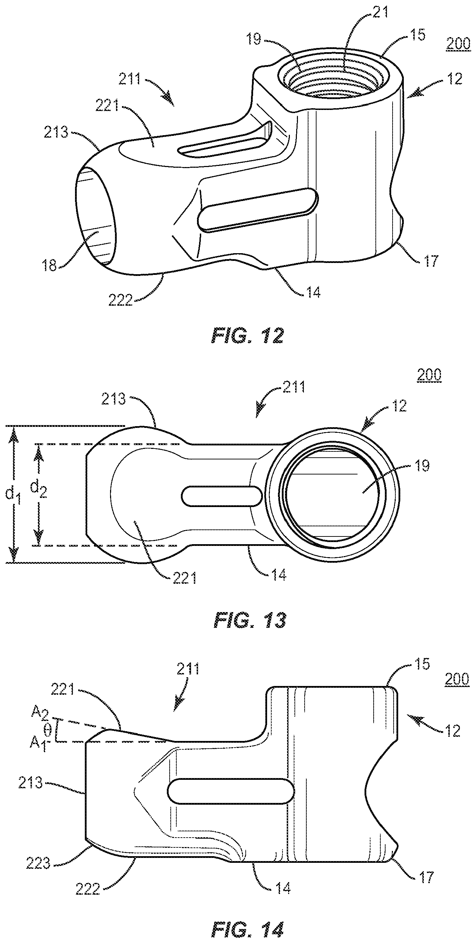

FIGs. 12-15 illustrate a connector 200 having a lordotic angle of Θ degrees, that is, axis Ar is 2 degrees above axis Ac. A first member 211 includes a first end 213 having an upper surface 221 and a lower surface 222. First end 213 maintains a circular shape with width d1 when observed down at an angle transverse to axis Ac. Upper surface 221 is also planar, but an axis of the planar surface A2 is offset from a normal axis A1 by angle Θ. This offset angle will define the angle that implant 20 offsets from axis Ac of channel 36. Lower surface 122 is arcuate in cross section transverse to axis Ac and configured to mate with a bottom of channel 36. To enable the offset of connector 200 to angle properly, lower surface 222 includes an offset area 223 that assists connector 200 to angle properly. The offset angle of offset area 223 is substantially equal to the offset angle of upper surface 221.

In operation, implant 20 is positioned within connector 200 and connector 200 is positioned within channel 36, similar to that shown in FIG. 6. The bulbous shape of first end 213 prevents movement along axis Ac of connector 200. Fixation element 16 is tightened onto implant 20 to affix implant 20 in connector 200. Fixation element 31 is tightened onto top surface 121 of connector 200. Since upper surface 221 is planar and offset at an angle 2 and offset area 223 is also at an angle Θ, fixation element 31 will prevent rotation of connector 100 and force lower surface 122 to securely mate with channel 36 with axis Ar of implant 20 offset from axis Ac of channel 36 by angle Θ. The bulbous shape of first end 213 mating with circular space 38 prevents any lateral movement and maintains connector 200 within channel 36.

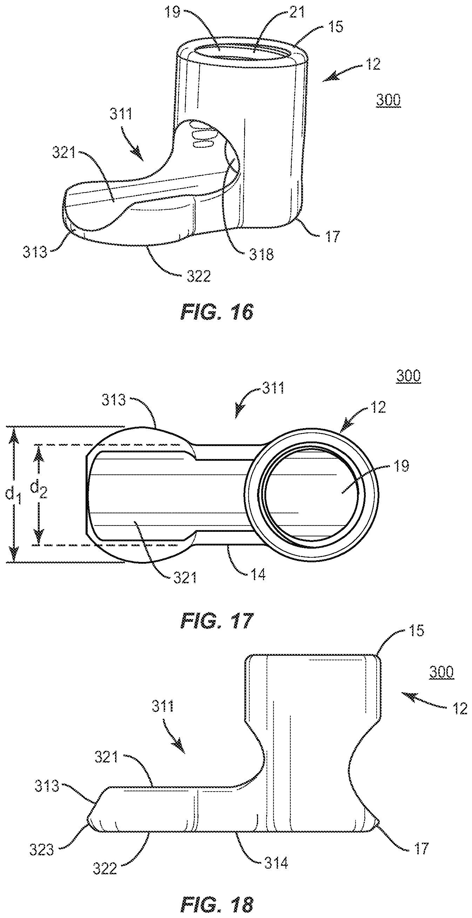

FIGs. 16-21 illustrate alternate embodiments of connectors in accordance with the principles of the present disclosure. In these embodiments, the first member is partially cut away and the cavity therein is partially exposed. Various offset angles can be obtained.

FIGs. 16-18 illustrate a connector 300 having a lordotic angle of 0 degrees, that is, axis Ar is parallel to axis Ac. A first member 311 includes a first end 313 having an upper surface 321 and a lower surface 322. First end 313 maintains a circular bulbous shape with width d when observed down at an angle transverse to axis Ac. As stated above, upper portions of first end 313 and a mid-section 314 of the connector has been removed exposing the cavity therein, while maintaining the cavity 18 in second end 17 in and about the area of cavity 19. The now upper surface 321, which was part of cavity 18, maintains its arcuate shape to mate with implant 20. Lower surface 322 is arcuate in cross section transverse to axis Ac and configured to mate with a bottom of channel 36 of bone screw 30.

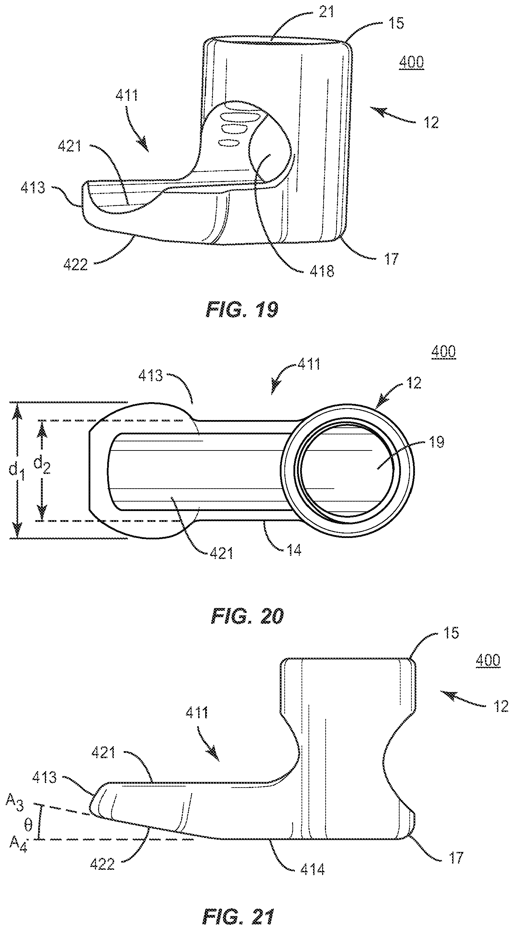

FIGs. 19-21 illustrate a connector 400 having a lordotic angle of Θ degrees, that is, axis Ar is Θ degrees above axis Ac. A first member 411 includes a first end 413 having an upper surface 421 and a lower surface 422. First end 413 maintains a circular shape with width d when observing down at an angle transverse to axis Ac. As stated above, upper portions of first end 413 and a mid-section 414 of the connector has been removed exposing the cavity therein, while maintaining the cavity 18 in second end 17 in and about the area of cavity 19. The now upper surface 421, which was part of cavity 18, maintains its arcuate shape to mate with implant 20. Lower surface 422 is arcuate in cross section transverse to axis Ac and configured to mate with a bottom of channel 36. To enable the offset of connector 400 to angle properly, an axis A3 of lower surface 422 is offset from a normal axis A4 by angle Θ. This offset angle defines the angle that implant 20 offsets from axis Ac of channel 36. Angle Θ may vary to induce other angles as prescribed by the surgeon and determined by the anatomy of the patient. Additionally, the angle may be reversed (or the connector 400 placed on the outside of the bone anchors) so that a kyphotic angle is produced when the implant is fixed in the receiver.

At the intersection of the lower surface 414 and the lower surface 422 a fulcrum is formed. The fulcrum is offset from the center of the receiver such that the fulcrum pivots the lower surface 422 to a position parallel to the longitudinal axis of the channel of the receiving portion when the implant connector is affixed to the bone anchor.

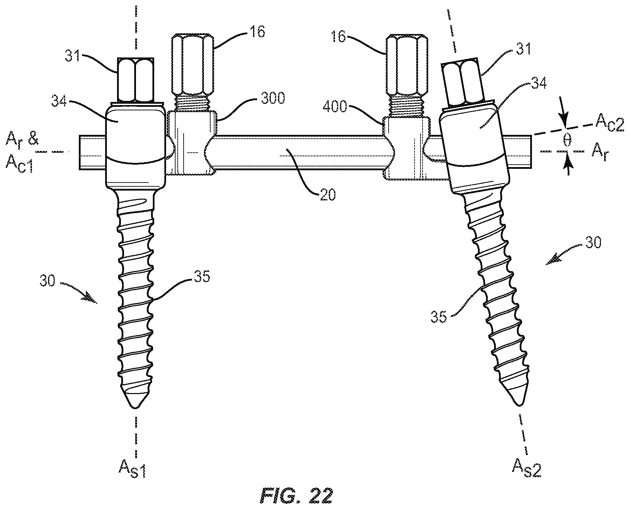

In operation, as shown in FIG. 22, implant 20 is positioned within connectors 300, 400 and connectors 300, 400 are positioned within channels 36 of bone screws 30, similar to that shown in FIG. 6. The bulbous shape of first ends 313 and 413 prevents movement along axis Ac of connectors 300, 400. Fixation elements 16 are tightened onto implant 20 to affix implant 20 in connectors 300, 400. Fixation elements 31 are tightened onto implant 20, as part of connectors 300, 400 have been removed. Fixation elements 31 force bottom surfaces 322, 422 to securely mate with respective channels 36. Connector 300 causes axis Ar of implant 20 to be in substantial alignment with axis Ac1 (i.e. 0 degrees) of channel 36 and connector 400 causes axis Ar of implant 20 to be offset from axis Ac2 of channel 36 by angle Θ. This creates an overall lordotic angle between axes As1 and As2 of angle Θ. As can be seen, if connector 300 is replaced with an angle connector (e.g. similar to connector 400) having an offset of angle β, the system can have an overall offset angle of Θ + β. As stated above, the bulbous shape of first ends 313, 413 mating with circular spaces 38 prevents any lateral movement and maintains connectors 300, 400 within channels 36 of their respective bone screws 30.

In addition, although the open ended variations of the connectors (i.e. connectors 300, 400) are illustrated in FIG. 22, all of the present embodiments of connectors (e.g. 10, 100, 200) can be used in various combinations to achieve differing offsets and configurations.

## Claims

1. An implant connector system, comprising:
a bone anchor (30) having a receiving portion defining a channel (36) extending along a longitudinal axis (Ac), the channel (36) including a circular space (38) and two opposing spaces (39) defined by inner edges (40) of opposing arms (34); and
an implant connector (10; 100; 200; 300; 400) comprising:
a first member (11; 111; 211; 311; 411) defining a first cavity (18; 318; 418) positioned along a longitudinal axis of the first member (11; 111; 211; 311; 411) and configured to receive an implant (20), the first member (11; 111; 211; 311; 411) includes a first end (13; 113; 213; 313; 413), a mid-section (14), and a second end (17), the first end (13; 113; 213; 313; 413) and the mid-section (14) forming an engagement member (13, 14; 113, 14; 213, 14; 313, 14; 413, 14) having a bulbous shape configured to engage with the circular space (38) and configured to prevent translational movement of the connector (10; 100, 200; 300; 400) along the longitudinal axis (Ac) of the receiving portion of the bone anchor (30), the first end (13; 113; 213; 313; 413) having a spherical surface configured to rotate the implant (20) relative to the longitudinal axis (Ac) of the receiving portion, wherein, when mated, the first end (13; 113; 213; 313; 413) fits within the circular space (38) and the mid-section (14) fits within one of the opposing spaces (39), the connector (10; 100; 200; 300; 400) being rotatable within the receiving portion due to the spherical shape of the surface of the first end (13; 113; 213; 313; 413); and
a second member (12) connected to the first member (11; 111; 211; 311; 411) along a first axis and defining a second cavity (19) positioned along a longitudinal axis of the second member (12) and transverse to the longitudinal axis of the first member (11; 111; 211; 311; 411) and configured to receive a fixation element (16) to affix the implant (20) within the first member (11; 111; 211; 311; 411), the second member (12) connected to the first member (11; 111; 211; 311; 411) substantially at the second end.

2. The implant connector system of claim 1, wherein the first (11; 111; 211; 311; 411) and second members (12) are monolithically formed.

3. The implant connector system of claim 1, further comprising an implant (20) wherein the implant (20) is a surgical rod.

4. The implant connector system of claim 1, wherein the bone anchor (30) includes a bone anchor fixation element (31) to affix the implant connector (10; 100; 200; 300; 400) within the receiving portion.

5. The implant connector system of claim 4, wherein the surface of the first end (13; 113; 213; 313; 413) of the first member (11; 111; 211; 311; 411) is substantially spherical in shape to permit rotation of the implant connector (10; 110; 210; 310; 410) within the receiving portion of the bone anchor (30) such that the longitudinal axis of the first member (11; 111; 211; 311; 411) is movable between a position substantially aligned with the longitudinal axis (Ac) defined along the receiving portion and a position transverse to the longitudinal axis (Ac) defined along the receiving portion.

6. The implant connector system of claim 5, wherein the bone anchor fixation element (31) is configured to affix the implant connector (10; 100; 200; 300; 400) at a position to which the implant connector (10; 100; 200; 300; 400) is rotated.

7. The implant connector system of claim 4, wherein the surface of the first end (213) of the first member (211) defines a planar surface (221) having an axis (A2) that is at an angle (θ) to the longitudinal axis (A1) of the first member.

8. The implant connector system of claim 7, wherein the bone anchor fixation element (31) is configured to affix the implant connector (200) within the receiving portion to maintain the axis (A2) of the surface (221) of the first end (213) substantially parallel to the axis (Ac) of the receiving portion.

9. The implant connector system of claim 4, wherein the first end (213) of the first member (211) includes an upper surface (221) and a lower surface (222) and the upper surface (221) defines a planar surface having an axis (A2) that is transverse to the longitudinal axis (A1) of the first member (211).

10. The implant connector system of claim 9, wherein the bone screw fixation element (31) is configured to affix the implant connector (200) within the channel (36) to maintain the axis (A2) of the upper surface (221) transverse to the axis (Ac) of the channel (36).

11. The implant connector system of claim 1, wherein the first member (311; 411) defines a channel adjoining the first cavity (318; 418) positioned along a longitudinal axis of the first member (311; 411), the channel and the cavity (318; 418) configured to receive the implant (20).

12. The implant connector system of claim 1, wherein the first end (413) of the first member (411) includes a lower surface (422) and a fulcrum at the intersection of the lower surface (422) of the first end (413) and a lower surface of a middle portion of the connector (400), the fulcrum offset from the center of the receiver such that the fulcrum pivots the lower surface (422) of the first end (413) to a position parallel to the longitudinal axis (Ac) of the channel (36) of the receiving portion when the implant connector (400) is affixed to the bone anchor (30).

13. The implant connector system of claim 12, wherein the lower surface (422) of the first end (413) is a planar surface, the angle (θ) between the lower surface (422) of the first end (413) and the lower surface of the middle portion defines an angle (θ) induced between the implant (20) and the bone anchor (30) such that the central axes of adjacent vertebra are angularly offset from each other.

14. The implant connector system of claim 12, wherein the bone anchor (30) includes a bone anchor fixation element (31) to affix the implant connector (400) within the receiving portion, the bone anchor fixation element (31) further configured to exert a force on the implant (20) within the receiver to pivot the implant (20) about the fulcrum.

## Patentansprüche

1. Ein Implantat-Verbindungssystem, aufweisend:
einen Knochenanker (30), welcher einen Aufnahmeabschnitt hat, der einen Kanal (36) definiert, welcher sich entlang einer Längsachse (Ac) erstreckt, wobei der Kanal (36) einen kreisförmigen Raum (38) und zwei einander gegenüberliegende Räume (39) aufweist, die von Innenrändern (40) einander gegenüberliegender Arme (34) definiert werden, und
ein Implantat-Verbindungsteil (10; 100; 200; 300; 400), aufweisend:
ein erstes Element (11; 111; 211; 311; 411), welches einen ersten Hohlraum (18; 318; 418) definiert, der entlang einer Längsachse des ersten Elements (11; 111; 211; 311; 411) positioniert ist und eingerichtet ist, um ein Implantat (20) aufzunehmen, wobei das erste Element (11; 111; 211; 311; 411) ein erstes Ende (13; 113; 213; 313; 413), einen Mittelabschnitt (14) und ein zweites Ende (17) aufweist, wobei das erste Ende (13; 113; 213; 313; 413) und der Mittelabschnitt (14) ein Eingriffselement (13, 14; 113, 14; 213, 14; 313, 14; 413, 14) bilden, das eine bauchige Form hat, die eingerichtet ist, um in den kreisförmigen Raum (38) einzugreifen und eingerichtet ist, um eine Translationsbewegung des Verbindungsteils (10; 100, 200; 300; 400) entlang der Längsachse (Ac) des Empfangsabschnitts des Knochenankers (30) zu verhindern, wobei das erste Ende (13; 113; 213; 313; 413) eine kugelförmige Fläche hat, die eingerichtet ist, um das Implantat (20) relativ zu der Längsachse (Ac) des Aufnahmeabschnitts zu rotieren, wobei, wenn zusammengefügt, das erste Ende (13; 113; 213; 313; 413) in den kreisförmigen Raum (38) passt und der Mittelabschnitt (14) in einen von den einander gegenüberliegenden Räumen (39) passt, wobei das Verbindungsteil (10; 100; 200; 300; 400) innerhalb des Aufnahmeabschnitts rotierbar ist aufgrund der Kugelform der Fläche des ersten Endes (13; 113; 213; 313; 413), und
ein zweites Element (12), welches mit dem ersten Element (11; 111; 211; 311; 411) entlang einer ersten Achse verbunden ist und einen zweiten Hohlraum (19) definiert, der entlang einer Längsachse des zweiten Elements (12) und quer zu der Längsachse des ersten Elements (11; 111; 211; 311; 411) positioniert ist und eingerichtet ist, um ein Fixierelement (16) aufzunehmen zum Fixieren des Implantats (20) innerhalb des ersten Elements (11; 111; 211; 311; 411), wobei das zweite Element (12) mit dem ersten Element (11; 111; 211; 311; 411) im Wesentlichen an dem zweiten Ende verbunden ist.

2. Implantat-Verbindungssystem gemäß Anspruch 1, wobei das erste Element (11; 111; 211; 311; 411) und das zweite Element (12) einstückig gebildet sind.

3. Implantat-Verbindungssystem gemäß Anspruch 1, ferner aufweisend ein Implantat (20), wobei das Implantat (20) ein chirurgischer Stab ist.

4. Implantat-Verbindungssystem gemäß Anspruch 1, wobei der Knochenanker (30) ein Knochenanker-Fixierteil (31) aufweist zum Fixieren des Implantat-Verbindungsteils (10; 100; 200; 300; 400) innerhalb des Aufnahmeabschnitts.

5. Implantat-Verbindungssystem gemäß Anspruch 4, wobei die Fläche des ersten Endes (13; 113; 213; 313; 413) des ersten Elements (11; 111; 211; 311; 411) im Wesentlichen kugelförmig ist, um ein Rotieren des Implantat-Verbindungsteils (10; 110; 210; 310; 410) innerhalb des Aufnahmeabschnitts des Knochenankers (30) zu erlauben, sodass die Längsachse des ersten Elements (11; 111; 211; 311; 411) bewegbar ist zwischen einer Position, die im Wesentlichen an der entlang des Aufnahmeabschnitts definierten Längsachse (Ac) ausgerichtet ist, und einer Position, die quer zu der entlang des Aufnahmeabschnitts definierten Längsachse (Ac) ist.

6. Implantat-Verbindungssystem gemäß Anspruch 5, wobei das Knochenanker-Fixierteil (31) eingerichtet ist, um das Implantat-Verbindungsteil (10; 100; 200; 300; 400) an einer Position zu fixieren, in die das Implantat-Verbindungsteil (10; 100; 200; 300; 400) rotiert wird.

7. Implantat-Verbindungssystem gemäß Anspruch 4, wobei die Fläche des ersten Endes (213) des ersten Elements (211) eine ebene Fläche (221) definiert, die eine Achse (A2) hat, welche in einem Winkel (θ) zu der Längsachse (A1) des ersten Elements ist.

8. Implantat-Verbindungssystem gemäß Anspruch 7, wobei das Knochenanker-Fixierteil (31) eingerichtet ist zum Fixieren des Implantat-Verbindungsteils (200) innerhalb des Aufnahmeabschnitts, um eine Achse (A2) der Fläche (221) des ersten Endes (213) im Wesentlichen parallel zu der Achse (Ac) des Aufnahmeabschnitts zu halten.

9. Implantat-Verbindungssystem gemäß Anspruch 4, wobei das erste Ende (213) des ersten Elements (211) eine obere Fläche (221) und eine untere Fläche (222) aufweist und die obere Fläche (221) eine ebene Fläche definiert, welche eine Achse (A2) hat, die quer zu der Längsachse (A1) des ersten Elements (211) ist.

10. Implantat-Verbindungssystem gemäß Anspruch 9, wobei das Knochenschraube-Fixierteil (31) eingerichtet ist zum Fixieren des Implantat-Verbindungsteils (200) innerhalb des Kanals (36), um die Achse (A2) der oberen Fläche (221) quer zu der Achse (Ac) des Kanals (36) zu halten.

11. Implantat-Verbindungssystem gemäß Anspruch 1, wobei das erste Element (311; 411) einen Kanal definiert, der an den ersten Hohlraum (318; 418), welcher entlang einer Längsachse des ersten Elements (311; 411) positioniert ist, angrenzt, wobei der Kanal und der Hohlraum (318; 418) eingerichtet sind, um das Implantat (20) aufzunehmen.

12. Implantat-Verbindungssystem gemäß Anspruch 1, wobei das erste Ende (413) des ersten Elements (411) eine untere Fläche (422) und einen Drehpunkt an der Schnittstelle der unteren Fläche (422) des ersten Endes (413) und einer unteren Fläche eines Mittelabschnitts des Verbindungsteils (400) aufweist, wobei der Drehpunkt versetzt ist von der Mitte des Aufnehmers, sodass der Drehpunkt die untere Fläche (422) des ersten Endes (413) in eine Position schwenkt, die parallel zu der Längsachse (Ac) des Kanals (36) des Aufnahmeabschnitts ist, wenn das Implantat-Verbindungsteil (400) an dem Knochenanker (30) fixiert wird.

13. Implantat-Verbindungssystem gemäß Anspruch 12, wobei die untere Fläche (422) des ersten Endes (413) eine ebene Fläche ist, wobei der Winkel (θ) zwischen der unteren Fläche (422) des ersten Endes (413) und der unteren Fläche des Mittelabschnitts einen Winkel (θ) definiert, der entsteht zwischen dem Implantat (20) und dem Knochenanker (30), sodass die Mittelachsen benachbarter Wirbel winklig voneinander versetzt sind.

14. Implantat-Verbindungssystem gemäß Anspruch 12, wobei der Knochenanker (30) ein Knochenanker-Fixierteil (31) aufweist zum Fixieren des Implantat-Verbindungsteils (400) innerhalb des Aufnahmeabschnitts, wobei das Knochenanker-Fixierteil (31) ferner eingerichtet ist, um eine Kraft auf das Implantat (20) in dem Aufnehmer auszuüben, um das Implantat (20) um den Drehpunkt zu schwenken.

## Revendications

1. Système de connecteur d'implant comprenant :
un ancrage osseux (30) ayant une partie de réception définissant un canal (36) s'étendant le long d'un axe longitudinal (Ac), le canal (36) comprenant un espace circulaire (38) et deux espaces opposés (39) définis par des bords internes (40) de bras opposés (34) ; et
un connecteur d'implant (10 ; 100 ; 200 ; 300 ; 400) comprenant :
un premier élément (11 ; 111 ; 211 ; 311 ; 411) définissant une première cavité (18 ; 318 ; 418) positionnée le long d'un axe longitudinal du premier élément (11 ; 111 ; 211 ; 311 ; 411) et configuré pour recevoir un implant (20), le premier élément (11 ; 111 ; 211 ; 311 ; 411) comprend une première extrémité (13 ; 113 ; 213; 313; 413), une section centrale (14) et une seconde extrémité (17), la première extrémité (13 ; 113 ; 213 ; 313 ; 413) et la section centrale (14) formant un élément de mise en prise (13, 14 ; 113, 14 ; 213, 14 ; 313, 14 ; 413, 14) ayant une forme renflée configurée pour se mettre en prise avec l'espace circulaire (38) et configuré pour empêcher le mouvement de translation du connecteur (10 ; 100, 200 ; 300 ; 400) le long de l'axe longitudinal (Ac) de la partie de réception de l'ancrage osseux (30), la première extrémité (13 ; 113 ; 213 ; 313 ; 413) ayant une surface sphérique configurée pour faire tourner l'implant (20) par rapport à l'axe longitudinal (Ac) de la partie de réception, dans lequel, lorsqu'elle est couplée, la première extrémité (13 ; 113 ; 213 ; 313 ; 413) épouse l'espace circulaire (38) et la section centrale (14) épouse l'intérieur de l'un des espaces opposés (39), le connecteur (10 ; 100 ; 200 ; 300 ; 400) pouvant tourner à l'intérieur de la partie de réception en raison de la forme sphérique de la surface de la première extrémité (13 ; 113 ; 213 ; 313 ; 413) ; et
un second élément (12) raccordé au premier élément (11 ; 111 ; 211 ; 311 ; 411) le long du premier axe et définissant une seconde cavité (19) positionnée le long d'un axe longitudinal du second élément (12) et de manière transversale par rapport à l'axe longitudinal du premier élément (11 ; 111 ; 211 ; 311 ; 411) et configuré pour recevoir un élément de fixation (16) afin de fixer l'implant (20) à l'intérieur du premier élément (11 ; 111 ; 211 ; 311 ; 411), le second élément (12) étant raccordé au premier élément (11 ; 111 ; 211 ; 311 ; 411) sensiblement au niveau de la seconde extrémité.

2. Système de connecteur d'implant selon la revendication 1, dans lequel les premier (11 ; 111 ; 211 ; 311 ; 411) et second éléments (12) sont formés de manière monolithique.

3. Système de connecteur d'implant selon la revendication 1, comprenant en outre un implant (20) dans lequel l'implant (20) est une tige chirurgicale.

4. Système de connecteur d'implant selon la revendication 1, dans lequel l'ancrage osseux (30) comprend un élément de fixation d'ancrage osseux (31) pour fixer le connecteur d'implant (10 ; 100 ; 200 ; 300 ; 400) à l'intérieur de la partie de réception.

5. Système de connecteur d'implant selon la revendication 4, dans lequel la surface de la première extrémité (13 ; 113 ; 213 ; 313 ; 413) du premier élément (11 ; 111 ; 211 ; 311 ; 411) a une forme sensiblement sphérique pour permettre la rotation du connecteur d'implant (10; 110; 210; 310; 410) à l'intérieur de la partie de réception de l'ancrage osseux (30) de sorte que l'axe longitudinal du premier élément (11 ; 111 ; 211 ; 311 ; 411) est mobile entre une position sensiblement alignée avec l'axe longitudinal (Ac) définie le long de la partie de réception et une position transversale par rapport à l'axe longitudinal (Ac) définie le long de la partie de réception.

6. Système de connecteur d'implant selon la revendication 5, dans lequel l'élément de fixation d'ancrage osseux (31) est configuré pour fixer le connecteur d'implant (10; 100; 200; 300; 400) dans une position dans laquelle le connecteur d'implant (10 ; 100 ; 200 ; 300 ; 400) tourne.

7. Système de connecteur d'implant selon la revendication 4, dans lequel la surface de la première extrémité (213) du premier élément (211) définit une surface planaire (221) ayant un axe (A2) situé à un angle (θ) par rapport à l'angle longitudinal (A1) du premier élément.

8. Système de connecteur d'implant selon la revendication 7, dans lequel l'élément de fixation d'ancrage osseux (31) est configuré pour fixer le connecteur d'implant (200) à l'intérieur de la partie de réception pour maintenir l'axe (A2) de la surface (221) de la première extrémité (213) sensiblement parallèle à l'axe (Ac) de la partie de réception.

9. Système de connecteur d'implant selon la revendication 4, dans lequel la première extrémité (213) du premier élément (211) comprend une surface supérieure (221) et une surface inférieure (222) et la surface supérieure (221) définit une surface planaire ayant un axe (A2) qui est transversal par rapport à l'axe longitudinal (A1) du premier élément (211).

10. Système de connecteur d'implant selon la revendication 9, dans lequel l'élément de fixation de vis à os (31) est configuré pour fixer le connecteur d'implant (200) à l'intérieur du canal (36) pour maintenir l'axe (A2) de la surface supérieure (221) transversal par rapport à l'axe (Ac) du canal (36).

11. Système de connecteur d'implant selon la revendication 1, dans lequel le premier élément (311 ; 411) définit un canal attenant à la première cavité (318 ; 418) positionné le long d'un axe longitudinal du premier élément (311 ; 411), le canal et la cavité (318 ; 418) étant configurés pour recevoir l'implant (20).

12. Système de connecteur d'implant selon la revendication 1, dans lequel la première extrémité (413) du premier élément (411) comprend une surface inférieure (422) et un pivot au niveau de l'intersection de la surface inférieure (422) de la première extrémité (413) et une surface inférieure d'une partie centrale du connecteur (400), le pivot étant décalé du centre du receveur de sorte que le pivot fait pivoter la surface inférieure (422) de la première extrémité (413) dans une position parallèle à l'axe longitudinal (Ac) du canal (36) de la partie de réception lorsque le connecteur d'implant (400) est fixé à l'ancrage osseux (30).

13. Système de connecteur d'implant selon la revendication 12, dans lequel la surface inférieure (422) de la première extrémité (413) est une surface planaire, l'angle (θ) entre la surface intérieure (422) de la première extrémité (413) et la surface inférieure de la partie centrale définit un angle (θ) induit entre l'implant (20) et l'ancrage osseux (30) de sorte que les axes centraux des vertèbres adjacentes sont décalés de manière angulaire les uns par rapport aux autres.

14. Système de connecteur d'implant selon la revendication 12, dans lequel l'ancrage osseux (30) comprend un élément de fixation d'ancrage osseux (31) pour fixer le connecteur d'implant (400) à l'intérieur de la partie de réception, l'élément de fixation d'ancrage osseux (31) étant en outre configuré pour exercer une force sur l'implant (20) à l'intérieur du receveur pour faire pivoter l'implant (20) autour du pivot.
